# EUROPEAN PATENT APPLICATION

(11) **EP 4 311 825 A1**
(43) Date of publication of application: **31.01.2024**
(21) Application number: 22187948.9
(22) Date of filing: 29.07.2022
(51) Int. Cl.: C07C 41/58, C07C 43/315, C07G 1/00, C08H 7/00, C08L 97/00

(54) **EXTRACTION PROCESS OF PHENOLIC LIGNIN MONOMERS FROM CRUDE LIQUID LIGNIN OIL (CLO)**

(71) Applicant: Vertoro B.V., 6167 RD Geleen (NL)
(72) Inventor: BOOT, Michael Dirk, 6167 RD Geleen (NL); HENSEN, Emiel Jan Maria, 6167 RD Geleen (NL); KOURIS, Panagiotis, 6167 RD Geleen (NL); HUANG, Xiaoming, 6167 RD Geleen (NL)
(74) Representative: IPecunia

(57) **Abstract**

The invention relates to methoxyphenol liquid-liquid extraction process comprising:
a) Subjecting crude liquid lignin oil (CLO) or dried crude liquid lignin oil (CLO), to a treatment with water allowing the precipitation of lignin oligomers obtaining a mixture;
b) Subjecting the obtained mixture to a filtration obtaining a filtrate comprising sugars and methoxyphenols like 4-(2,2-dimethoxyethyl)-2-methoxyphenol;
c) Subjecting the filtrate comprising sugars and 4-(2,2-dimethoxyethyl)-2-methoxyphenol to a treatment with organic solvent to separate the 4-(2,2-dimethoxyethyl)-2-methoxyphenol from the sugars.

## Description

The invention relates to an extraction process of phenolic lignin monomers from crude liquid lignin oil (CLO).

Lignocellulosic biomass is the most abundantly available raw material on the Earth for the production of biofuels. It is composed of two kinds of carbohydrate polymers, cellulose in a quantity from 35% to 50% (composed exclusively of hexoses) and hemicellulose in a quantity from 20% to 30% (essentially consisting of pentoses), and an aromatic-rich polymer called lignin in a quantity from 15% to 25%. The sugar monomers of cellulose and hemicellulose are covalently bound to lignin making the separation of these components of the lignocellulosic materials very challenging.

Lignocellulosic materials, in the form of wood fuel, has a long history as a source of energy and the interest in it as a precursor to liquid fuels has increased enormously especially because several lignocellulosic materials as annual crop residue, can be a carbon neutral source of energy in the long run.

To extract the fermentable sugars used for the production of ethanol or other lignocellulose-derived fuels as butanol, one must first disconnect the celluloses from the lignin, and then use acid or enzymatic methods to hydrolyze the newly freed celluloses to break them down into simple monosaccharides.

Lignocellulosic materials have also been considered in the production of biocomposites materials such as particle panels, wood-plastic composites, and cement/geopolymer wood composites.

Biocomposites materials produced with lignocellulosic materials as alternative to conventional materials, are very attractive because are renewable and cheaper but also because they fit perfectly into the new policy of the "cascade utilization" of the resources.

Typically, the separation of cellulose, hemicellulose and lignin of the lignocellulosic material is performed in a reactor vessel.

WO 2021/064047 describes lignocellulosic feedstock such as hardwood or softwood in the form of chips or sawdust provided in a reactor vessel and treated with a polar organic solvent by providing the polar organic solvent into the reactor vessel to solve the lignocellulosic material. Also, an inorganic acid is provided in the reactor vessel to act as a reactant, cleaving lignin-carbohydrate linkages and releasing lignin from the lignocellulosic matrix of the lignocellulosic material. WO 2021/064047 fractionation method provides a crude liquid lignin oil (CLO) which consists mainly of sugars, like methylated C5 and C6 sugars, lignin oligomers in the presence of a low amount of solvent, preferably methanol or ethanol.. Crude liquid lignin oil (CLO) is an intermediate that can be used for the production of fuels and chemicals.

We discovered that next to methylated C5 and C6 sugars also phenolic lignin monomers like (alky)-methoxyphenol compounds are present in the CLO, which can have value in different applications. Examples of such methoxyphenols are the compound 4-(2,2-dimethoxyethyl)-2-methoxyphenol, vanillin and dihydroeugenol.

A major problem now is to separated the different components in the CLO, for example to separate phenolic lignin monomers, such as methoxy phenol components like for example the component 4-(2,2-dimethoxyethyl)-2-methoxyphenol from the sugars.

To allow a further commercialization of the methoxyphenol components like for example compound 4-(2,2-dimethoxyethyl)-2-methoxyphenol, there is a need of a method to separate the compounds like 4-(2,2-dimethoxyethyl)-2-methoxyphenolfrom the sugars.

The aim of the invention is to develop an extraction process to separate the methoxy phenol components like 4-(2,2-dimethoxyethyl)-2-methoxyphenol from the sugars.

After extensive studies, the inventors solved the above-mentioned problem and developed an efficient extraction process to separate the methoxy phenol components like 4-(2,2-dimethoxyethyl)-2-methoxyphenol from the sugars.

### Summary of the invention

The invention relates to a liquid-liquid extraction process comprising:
a) Subjecting crude liquid lignin oil (CLO) or dried crude liquid lignin oil (CLO), to a treatment with water allowing the precipitation of lignin oligomers obtaining a mixture;
b) Subjecting the obtained mixture to a filtration obtaining a filtrate comprising sugars and 4-(2,2-dimethoxyethyl)-2-methoxyphenol;
c) Subjecting the filtrate comprising sugars and 4-(2,2-dimethoxyethyl)-2-methoxyphenol to a treatment with organic solvent to separate the 4-(2,2-dimethoxyethyl)-2-methoxyphenol from the sugars;
wherein the organic solvent is used in an amount (weight) that ranges between 1:5 and 5:1 relative to the amount (weight) of filtrate of step c.

### Figures

Figure 1. depicts the performance of the different organic solvents used in the extraction process of the invention.
Figure 2. depicts the purity of the 4-(2,2-dimethoxyethyl)-2-methoxyphenol obtained with the extraction process of the invention performed using different organic solvents.

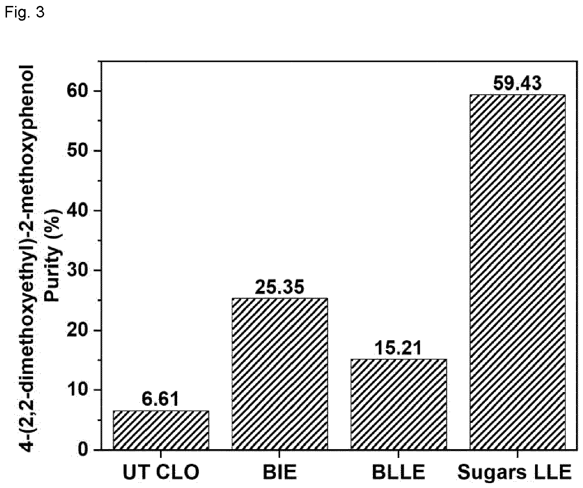

Figure 3. depicts the purity of the 4-(2,2-dimethoxyethyl)-2-methoxyphenol obtained with the extraction process of the invention

### Detailed description of the invention

The present invention relates to an extraction process of methoxyphenol components like 4-(2,2-dimethoxyethyl)-2-methoxyphenol from crude liquid lignin oil (CLO).

In particular, the invention relates to a liquid-liquid extraction process comprising:
a) Subjecting crude liquid lignin oil (CLO) or dried crude liquid lignin oil (CLO), to a treatment with water allowing the precipitation of lignin oligomers obtaining a mixture;
b) Subjecting the obtained mixture to a filtration obtaining a filtrate comprising sugars and 4-(2,2-dimethoxyethyl)-2-methoxyphenol;
c) Subjecting the filtrate comprising sugars and 4-(2,2-dimethoxyethyl)-2-methoxyphenol to a treatment with organic solvent to separate the 4-(2,2-dimethoxyethyl)-2-methoxyphenol from the sugars;
wherein the organic solvent is used in an amount (weight) that ranges between 1:5 and 5:1 relative to the amount (weight) of filtrate of step c.

Preferably the amount (weight) of organic solvent used ranges between 1:2 and 2:1 relative to the amount (weight) of filtrate of step c.

Lignin-rich solid feedstock is based on lignocellulosic biomass feedstock pre-treatment processes, such as acidic pulping, alkaline pulping (either Kraft or Soda), Bergius-Rheinau process, steam explosion, organosolv pulping, (dilute) acid based hydrolysis, fraction processes based on Ionic Liquids (ILs), liquid salts (e.g., zinc chloride hydrate) or Deep eutectic solvents (DES), superheated or supercritical steam.

When lignin-rich solid feedstock is dispersed in a polar organic solvent and subjected to a mild depolymerization process (solvolysis reaction), a crude liquid lignin oil (CLO) can be obtained.

For the purposes of the present invention, the term crude liquid lignin oil (CLO) refers to a mixture of lignin oligomers, methylated sugars and phenolic components, and a polar organic solvent in 1:2.5 to 1:1 w/v ratio. Preferably the polar organic solvent in the CLO is chosen from methanol and ethanol, preferably methanol.

For the purposes of the present invention, the term dried crude liquid lignin oil (CLO) refers to a mixture of lignin oligomers, methylated sugars and phenolic components, and a polar organic solvent in 1:1 to 1:0 w/v ratio, which is obtained by evaporating (all or a part of) the solvent of the CLO. Preferably the dried CLO contains less than 10 wt% of methanol, more preferably between 0.5 and 5 wt% methanol.

The crude liquid lignin oil (CLO) consists mainly of sugars, like C5 and C6 methylated sugars, lignin oligomers and organic molecules like methoxyphenol components, which are obtained when a lignocellulosic solid feedstock is subjected to a depolymerisation process. The mild depolymerisation process involves the cleavage of the (relatively) weak ether linkages of the lignin-rich solid feedstock and break down of lignin into lignin oligomers and sugars, mainly methylated sugars. Examples of methylated sugars are methyl-pentopyranoside, methyl-D-gluconpyranoside, methyl-D-xylopyranoside, methyl 3-O-acetylpentopyranoside, dimethyl-4-O-methyl-hexanopyroside, or a mixture thereof.

By tuning the process conditions in the reactor (lignin to solvent ratio, temperature, pressure residence time) the ratio between the CLO constituents can be controlled. Typically, the crude liquid lignin oil (CLO) comprising lignin oligomers and sugars has a ratio (w/w) of lignin oligomers to sugars ranging between 1:1 and 2.5:1.

Beside the sugars, mainly methylated sugars, and lignin oligomers, a further important constituent is the compound 4-(2,2-dimethoxyethyl)-2-methoxyphenol.

The compound 4-(2,2-dimethoxyethyl)-2-methoxyphenol is a very interesting compound because it may be used in different application; for example, it may be used in food industry.

Typically, the amount of 4-(2,2-dimethoxyethyl)-2-methoxyphenol in the sugars is in a range of from 1.5 to 10 wt.% relative to the crude liquid lignin oil (CLO), preferably between 2 to 6 wt%.

Subjecting the crude liquid lignin oil (CLO) to the process as described above, the methoxyphenol components like 4-(2,2-dimethoxyethyl)-2-methoxyphenol can be easily separated form the sugars and ready for further applications.

As will be understood by workers skilled in the art, the wording "liquid-liquid extraction" refers to a separation technique which allows the transfer of a compound from one liquid phase to another liquid phase.

As will be understood by workers skilled in the art, the crude liquid lignin oil (CLO) to be used in the current invention may be obtained with any methods know in the art. A preferred crude liquid lignin oil (CLO) to be used in the current invention is obtained by the method disclosed in WO 2021/064047.

Alternatively, the crude liquid lignin oil (CLO) to be used in the current invention may be a dried crude liquid lignin oil (CLO).

As will be understood by workers skilled in the art, the dried crude liquid lignin oil (CLO) may be obtained by subjecting the crude liquid lignin oil (CLO) to a drying process. As will be understood by workers skilled in the art, any drying process can be used to obtain a dried crude liquid lignin oil (CLO).

Examples of drying process are subjecting the crude liquid lignin oil (CLO) in an oven, a rotary vacuum evaporation, distillation, vacuum distillation, multi stage evaporation.

The crude liquid lignin oil (CLO) or dried crude liquid lignin oil (CLO), to be used in the current invention is subjected to a treatment with water. Typically, the crude liquid lignin oil (CLO) or dried crude liquid lignin oil (CLO), is mixed under stirring with water allowing the precipitation of the insoluble lignin oligomers providing a mixture comprising precipitated insoluble lignin oligomers, sugars, mainly methylated sugars, solubilized in water and 4-(2,2-dimethoxyethyl)-2-methoxyphenol also solubilized in water.

The v/v ratio between water used relative to CLO ranges between 0.5:1 and 5:1 v/v, preferably between 0.9:1 and 1:4 v/v, more preferably between 1:1 and 1:3 v/v.

After, the obtained mixture is subjected to a filtration. As will be understood by workers skilled in the art, the filtration may be performed with any filtration methods know in the art. Examples of filtration methods are decanter, centrifuge, filter press.

When the mixture is subjected to a filtration, the precipitated insoluble lignin oligomers are separated from the soluble sugars, mainly methylated sugars, and 4-(2,2-dimethoxyethyl)-2-methoxyphenol. The aqueous filtrate therefore comprises the soluble sugars, mainly methylated sugars, and 4-(2,2-dimethoxyethyl)-2-methoxyphenol.

Optionally, the aqueous filtrate is dried providing a viscous product which comprises sugars, mainly methylated sugars, and 4-(2,2-dimethoxyethyl)-2-methoxyphenol. As will be understood by workers skilled in the art, any drying process, as by using a rotary evaporator and/or by an overnight drying in a vacuum oven, can be used to dry the aqueous filtrate.

Successively, the filtrate or the viscous product comprising sugars, mainly methylated sugars, and 4-(2,2-dimethoxyethyl)-2-methoxyphenol is treated with organic solvent. This treatment is performed mixing the filtrate or the viscous product comprising sugars, mainly methylated sugars, and 4-(2,2-dimethoxyethyl)-2-methoxyphenol with organic solvent.

For the purposes of the present invention, the term "mixing" refers to any mixing, shaking, dispersing, agitation or emulsification methods.

Examples of mixing methods are stirred batch reactors, Nauta mixers or by using a separation funnel.

The mixing is performed for a sufficient time.

For the purposes of the present invention, the term "sufficient time" refers to the time needed to complete the extraction of 4-(2,2-dimethoxyethyl)-2-methoxyphenol from the filtrate, or preferably at least 99 wt% of the 4-(2,2-dimethoxyethyl)-2-methoxyphenol from the filtrate.

In a specific embodiment of the current invention, the filtrate or the viscous product comprising sugars, mainly methylated sugars, and 4-(2,2-dimethoxyethyl)-2-methoxyphenol is introduced in a separatory funnel to which the organic solvent is added. The two phases system is shaken vigorously for several minutes and let it rest to have again a separation of the two phases.

The organic phase comprises the 4-(2,2-dimethoxyethyl)-2-methoxyphenol while the water phase comprises the sugars, mainly methylated sugars. Optionally the 4-(2,2-dimethoxyethyl)-2-methoxyphenol may be isolated from the organic solvent.

As will be understood by workers skilled in the art, any isolation method can be used to isolate the 4-(2,2-dimethoxyethyl)-2-methoxyphenol.

The quantity of organic solvent to be used in the current invention is in an amount that ranges between 1:5 and 5:1 relative to the amount (weight) of filtrate of step c.

Preferably the amount (weight) of organic solvent used ranges between 1:2 and 2:1 relative to the amount (weight) of filtrate of step c.

The organic solvent to be used in the current invention is preferably selected from hexane, petroleum ether, chloroform, ethyl acetate, toluene and benzene.

More preferably, the organic solvent to be used in the current invention is selected from ethyl acetate, toluene and benzene. The use of ethylacetate, toluene and benzene increases the purity and overall yield of the recovery of 4-(2,2-dimethoxyethyl)-2-methoxyphenol. More preferably, the organic solvent to be used in the current invention is toluene, which gives the highest yield of 4-(2,2-dimethoxyethyl)-2-methoxyphenol.

As will be understood by workers skilled in the art the current invention allows the separation of lignin oligomers, sugars, mainly methylated sugars, and methoxyphenols like 4-(2,2-dimethoxyethyl)-2-methoxyphenol present in the crude liquid lignin oil (CLO) or dried crude liquid lignin oil (CLO).

The 4-(2,2-dimethoxyethyl)-2-methoxyphenol extracted by the process as described above, is obtained in an amount that ranges between 1 and 10 wt.%, preferably between 2 and 5 wt.%, relative to the amount of CLO..

Another embodiment of the invention relates to a 4-(2,2-dimethoxyethyl)-2-methoxyphenol obtained by the process as described above.

The 4-(2,2-dimethoxyethyl)-2-methoxyphenol obtained by the process as described above
has a purity higher than 40 wt%, preferably higher than 60wt%, more preferably higher than 80 wt%.

### Examples

### Example 1

### Extraction of 4-(2,2-dimethoxyethyl)-2-methoxyphenol from crude liquid lignin oil (CLO)

Crude liquid lignin oil (CLO) (40ml) is mixed with 120ml of demi water, followed by vigorous stirring and letting the lignin oligomers to precipitate. The resulting mixture was filtered through a gooch funnel filter. The aqueous phase was evaporated in the rotary evaporator, followed by overnight drying in a vacuum oven. The resulting viscous product constitutes the sugars, whereas the solids on top of the filter are the lignin oligomers.

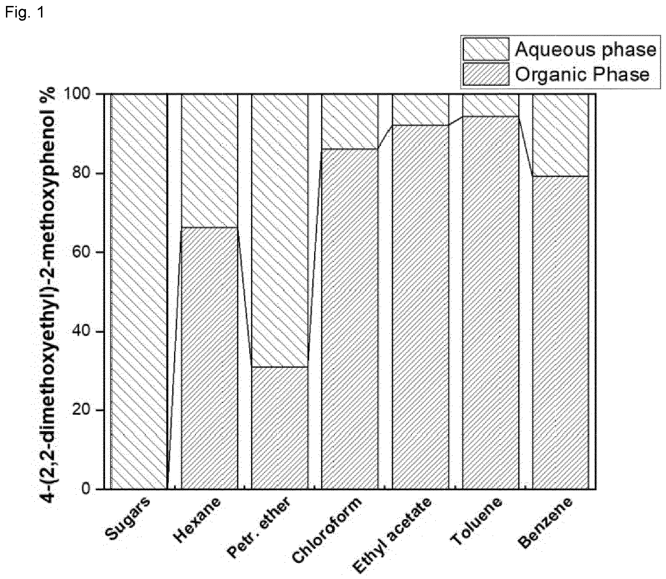

The resulting viscous product is introduced in an 100ml-separatory funnel. 1:1 (vol) organic solvent (hexane or petroleum ether or chloroform or ethyl acetate or toluene or benzene) is added in a 10ml aqueous solution of 1 % wt. dissolved sugars. The mixture is vigorously shaken for 4 minutes with frequent release of the vapor pressure and afterwards let the phases to separate. Separated phases were put in pre-weighted 10ml-round bottom flasks, followed by solvent removal and overnight drying in a vacuum oven. Final product was dissolved in methanol and analyzed in GC/GC-MS with the addition of n-dodecane as internal standard. Figure 1. Shows the performance of the different organic solvents used.

### Example 2

### Purity of 4-(2,2-dimethoxyethyl)-2-methoxyphenol extracted from crude liquid lignin oil (CLO)

The qualitative results of the extraction were performed as % purity in the organic phase.

The purity is given based on relative abundance from GC-MS peak area.

Spectra of the corresponding analyses from the tested extraction systems indicate variations in the chemical profile of the extractives in different solvents.

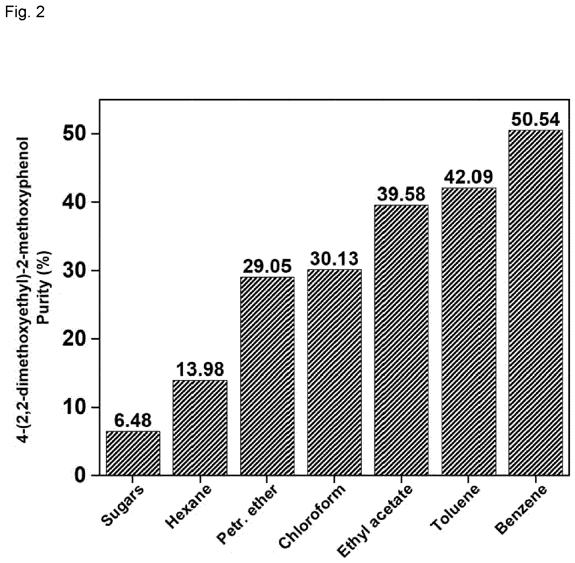

Figure 2. Shows the purity of the 4-(2,2-dimethoxyethyl)-2-methoxyphenol obtained with the extraction process of the invention performed using the different organic solvents (hexane, petroleum ether, chloroform, ethyl acetate, toluene and benzene).

## Claims

1. A liquid-liquid extraction process comprising:
a) Subjecting crude liquid lignin oil (CLO) or dried crude liquid lignin oil (CLO), to a treatment with water allowing the precipitation of lignin oligomers obtaining a mixture;
b) Subjecting the obtained mixture to a filtration obtaining a filtrate comprising sugars and methoxyphenols;
c) Subjecting the filtrate comprising sugars and the methoxyphenols to a treatment with organic solvent to separate the methoxyphenols from the sugars;
wherein the organic solvent is used in an amount (weight) that ranges between 1:5 and 5:1 relative to amount (weight) of filtrate of step c.

2. The extraction process according to claim 1, wherein the organic solvent is selected from hexane, petroleum ether, chloroform, ethyl acetate, toluene and benzene.

3. The extraction process according to claim 1, wherein the organic solvent is selected from ethyl acetate, toluene and benzene.

4. The extraction process according to any one of claims 1-3, wherein crude liquid lignin oil (CLO) refers to a homogeneous mixture of lignin oligomers, methylated sugars and phenolic components, and a polar organic solvent in 1:2.5 to 1:1 w/v ratio, wherein preferably the polar organic solvent is chosen from methanol and ethanol, preferably methanol.

5. The extraction process according to any one of claims 1-4, wherein the methoxyphenols comprise 4-(2,2-dimethoxyethyl)-2-methoxyphenol.

6. The extraction process according to claims 1 to 5, wherein the treatment with water in step b is performed by mixing under stirring with water.

7. The extraction process according to claims 1 to 6, wherein the water is used in an amount that ranges between 0.5:1 and 5:1 v/v, preferably between 0.9:1 and 1:4 v/v, more preferably between 1:1 and 1:3 v/v.% relative to the crude liquid lignin oil (CLO) or dried crude liquid lignin oil (CLO).

8. The extraction process according to claims 1 to 7, wherein the treatment with organic solvent is performed by mixing the filtrate with organic solvent.
